# FASCICULE DE BREVET EUROPEEN

(11) **EP 4 452 288 B1**
(45) Date de publication et mention de la délivrance du brevet: **14.01.2026**
(21) Numéro de dépôt: 22847593.5
(22) Date de dépôt: 20.12.2022
(51) Int. Cl.: A61K 35/747, A23L 33/135, A61K 31/519, A61K 31/7076, A61K 31/737, A61K 9/19, A61K 36/064, A61K 38/06, A61P 31/10, A61K 31/122, A61P 15/02, C12R 1/225, A61K 9/00, C12N 1/20

(54) **UTILISATION D'UNE SOUCHE BACTÉRIENNE LIMOSILACTOBACILLUS FERMENTUM SEULE OU EN ASSOCIATION AVEC SACCHAROMYCES CEREVISIAE POUR PRÉVENIR ET/OU TRAITER LES CANDIDOSES VAGINALES**
VERWENDUNG EINES LIMOSILACTOBACILLUS FERMENTUM BAKTERIENSTAMM ALLEIN ODER IN KOMBINATION MIT SACCHAROMYCES CEREVISIAE ZUR VORBEUGUNG UND/ODER BEHANDLUNG VON VAGINALER CANDIDOSE
USE OF A LIMOSILACTOBACILLUS FERMENTUM BACTERIAL STRAIN ALONE OR IN COMBINATION WITH SACCHAROMYCES CEREVISIAE FOR PREVENTING AND/OR TREATING VAGINAL CANDIDIASIS

(30) Priorité: 21.12.2021 FR 2114188
(43) Date de publication de la demande: 30.10.2024
(73) Titulaire: Lesaffre et Compagnie, 75001 Paris (FR)
(72) Inventeur: BALLET, Nathalie, 59700 MARC EN BAROEUL (FR); BOYER, Mickaël, 59700 MARCQ EN BAROEUL (FR); JIMENEZ CALDERON, Luciana, 59000 LILLE (FR); RODRIGUEZ, Bertrand, 59130 LAMBERSART (FR)
(74) Mandataire: Plasseraud IP
(86) Numéro de dépôt international: PCT/FR2022/052438
(87) Numéro de publication internationale: WO 2023/118729

(56) Documents cités:
- WO-A1-2014/009656
- US-A1- 2020 113 954
- KANG CHANG-HO ET AL: "In vitro probiotic properties of vaginalLactobacillus fermentumMG901 andLactobacillus plantarumMG989 againstCandida albicans", EUROPEAN JOURNAL OF OBSTETRICS & GYNECOLOGY AND REPRODUCTIVE BIOLOGY, ELSEVIER IRELAND LTD, IE, vol. 228, 9 July 2018 (2018-07-09), pages 232 - 237, XP085454261, ISSN: 0301-2115, DOI: 10.1016/J.EJOGRB.2018.07.005
- MACALPINE JESSIE ET AL: "A small molecule produced by Lactobacillus species blocks Candida albicans filamentation by inhibiting a DYRK1-family kinase", vol. 12, no. 1, 22 October 2021 (2021-10-22), XP055939829, Retrieved from the Internet <URL:https://www.nature.com/articles/s41467-021-26390-w.pdf> DOI: 10.1038/s41467-021-26390-w
- FALAGAS M E ET AL: "Probiotics for prevention of recurrent vulvovaginal candidiasis: a review", JOURNAL OF ANTIMICROBIAL CHEMOTHERAPY, OXFORD UNIVERSITY PRESS, GB, vol. 58, 21 June 2006 (2006-06-21), pages 266 - 272, XP003013958, ISSN: 0305-7453, DOI: 10.1093/JAC/DKL246

## Description

### Domaine technique

La présente invention relève du domaine de la santé vaginale. Elle concerne notamment l'utilisation de nouvelles souches bactériennes *Limosilactobacillus fermentum,* dans la prévention et/ou le traitement des candidoses vaginales. L'invention concerne également l'utilisation de ces nouvelles souches bactériennes *Limosilactobacillus fermentum* en association avec *Saccharomyces cerevisiae* dans la prévention et/ou le traitement des candidoses vaginales.

### Technique antérieure

Les candidoses vaginales ou vulvo-vaginales (encore appelées mycoses vaginales ou vulvo-vaginales) sont provoquées principalement par une infection par des champignons pathogènes, à savoir des levures du type *Candida albicans* (d'où le nom de candidose) au niveau de la zone intime des femmes. *Candida albicans,* naturellement présent dans le microbiote vaginal, peut proliférer anormalement et entraîner le développement de la candidose vaginale. Les candidoses ou mycoses vaginales entraînent des démangeaisons ainsi que l'inflammation de la vulve et du vagin.

Les mycoses vaginales sont considérées à ce jour comme un important problème de santé public et constituent la deuxième cause d'infection vaginale après les vaginites bactériennes. Il est estimé que 75% des femmes développent un jour ou l'autre une mycose vaginale. Environ 50% d'entre elles subissent au moins 2 à 3 épisodes de mycoses vaginales au cours de leur vie. 10 à 20% des femmes souffrent de mycoses vaginales récidivantes avec en moyenne 4 épisodes par an.

Les traitements antimycotiques habituellement utilisés à ce jour sont basés sur l'emploi de molécules chimiques, principalement des dérivés azoles tels que par exemple fluconazole, kétoconazole, éconazole, miconazole ou clotrimazole, administrés généralement sous forme de crème ou d'ovule à introduire dans le vagin. Dans certains cas, un traitement par voie orale vient compléter le traitement par voie locale.

Cependant, un des problèmes majeurs de ce type de traitement est que, lors de mycoses vaginales à répétition, le champignon pathogène, en particulier *Candida albicans,* peut développer des résistances aux traitements antimycotiques existants.

Un autre problème dans l'utilisation de dérivés azoles pour soigner les mycoses est la sensation d'irritation et de cuisson au niveau de la vulve.

Afin de proposer des alternatives aux traitements à base de dérivés azoles, des probiotiques ont été décrits dans la littérature.

En 2001, les probiotiques ont été définis par l'Organisation mondiale de la santé (OMS) comme des « micro-organismes vivants qui, lorsqu'ils sont ingérés en quantité suffisante, exercent des effets positifs sur la santé, au-delà des effets nutritionnels traditionnels ».

Le document PCT/SE2006/001311 décrit ainsi l'utilisation d'une souche de *Lactobacillus fermentum* « Ess-1 », déposée auprès de la « Deutsche Sammlung von Mikroorganismen und Zellkulturen GmbH » sous le numéro « DSM17851 », dans la prévention et/ou le traitement de candidoses vaginales.

Le document PCT/FR2013/051643 décrit l'utilisation d'une souche *Saccharomyces cerevisiae* déposée auprès de la CNCM sous le numéro I-3856 dans la prévention et/ou le traitement des mycoses vaginales, et en particulier des candidoses vaginales.

Les probiotiques ne présentent pas les inconvénients susmentionnés des traitements chimiques (phénomène de résistance, effets secondaires tels qu'irritation, brûlure) tout en ayant une efficacité au moins comparable auxdits traitements. Les probiotiques représentent ainsi des solutions alternatives naturelles, sans effets indésirables, aux molécules chimiques.

### Résumé

Un des objectifs de la présente invention est donc de proposer de nouveaux probiotiques pour prévenir et/ou traiter les candidoses vaginales.

Les présents inventeurs ont trouvé de manière surprenante que l'utilisation d'au moins une souche bactérienne *Limosilactobacillus fermentum* déposée le 17 novembre 2021 auprès de la CNCM (Collection Nationale de Cultures de Microorganismes, 25, rue du Docteur Roux, 75724 Paris cedex 15, France) sous le numéro I-5777 ou sous le numéro I-5778 permettait d'atteindre cet objectif.

Un objet de l'invention réside ainsi dans une souche *Limosilactobacillus fermentum* choisie parmi la souche déposée auprès de la CNCM le 17 novembre 2021 sous le numéro I-5777 ou sous le numéro I-5778.

Il est rappelé que l'effet probiotique d'une souche donnée, qu'il s'agisse d'une souche de bactérie ou d'une souche de levure, est spécifique de cette souche, et non du genre et de l'espèce considéré.

Un autre objet de l'invention réside dans une souche *Limosilactobacillus fermentum* choisie parmi la souche déposée auprès de la CNCM le 17 novembre 2021 sous le numéro I-5777 ou sous le numéro I-5778, pour son utilisation dans la prévention et/ou le traitement des candidoses vaginales.

La souche *Limosilactobacillus fermentum* est une souche de bactérie ou souche bactérienne. L'expression « souche de bactérie » désigne une population relativement homogène de cellules de bactérie.

Une souche de bactérie est obtenue à partir de l'isolement d'un clone, un clone étant une population de cellules obtenue à partir d'une seule cellule de bactérie.

La présente invention porte également sur la bactérie obtenue par culture de la souche *Limosilactobacillus fermentum* choisie parmi la souche déposée auprès de la CNCM le 17 novembre 2021 sous le numéro I-5777 ou sous le numéro I-5778, pour son utilisation dans la prévention et/ou le traitement des candidoses vaginales, pour son utilisation dans la prévention et/ou le traitement des candidoses vaginales.

Dans la présente demande, le terme « bactérie » est employé au singulier mais il pourrait aussi être employé au pluriel. La « bactérie » désigne ainsi la ou les bactéries obtenues par culture de la souche *Limosilactobacillus fermentum* telle que ci-dessus définie. On utilisera indifféremment, dans la présente demande, les termes « bactérie obtenue par culture de la souche *Limosilactobacillus fermentum* déposée à la CNCM le 17 novembre 2021 sous le numéro I-5777 », « bactérie obtenue par culture de la souche I-5777 » et « bactérie I-5777 », ces trois termes ayant la même signification que celle donnée ci-dessus pour le terme « bactérie ».

De même, on utilisera indifféremment, dans la présente demande, les termes « bactérie obtenue par culture de la souche *Limosilactobacillus fermentum* déposée à la CNCM le 17 novembre 2021 sous le numéro I-5778", « bactérie obtenue par culture de la souche I-5778 » et « bactérie I-5778 », ces trois termes ayant la même signification telle que donnée ci-dessus.

Les termes « souche bactérienne », « souche de bactérie », « souche *Limosilactobacillus fermentum* » désignent, au sens de la présente demande, la souche I-5777 et/ou la souche I-5778.

La bactérie *Limosilactobacillus fermentum* de l'invention désigne, au sens de la présente demande, la bactérie I-5777 et/ou la bactérie I-5778.

A titre indicatif, la bactérie *Limosilactobacillus fermentum* de l'invention peut être obtenue par culture de la souche I-5777 ou de la souche I-5778, à 37°C, sans agitation, dans un milieu de culture « bouillon MRS » (« Man Rogosa Sharpe ») (Biomerieux - AEB140652) à un pH de 6,5 pendant une nuit.

La bactérie *Limosilactobacillus fermentum* peut cependant également être obtenue par un procédé à l'échelle industrielle qui comprend les étapes suivantes :
- culture de la souche de bactérie dans un milieu optimal pour la production de biomasse,
- séparation par centrifugation de la bactérie ainsi produite de son milieu de culture, pour obtenir une biomasse concentrée en crème contenant de 10 à 20% de matière sèche, ladite biomasse concentrée étant appelée « centrifugat »,
- congélation du centrifugat, de préférence avec de l'azote liquide,
- éventuellement conservation à - 80°C du centrifugat congelé pendant une durée pouvant aller jusqu'à un an,
- séchage par lyophilisation du centrifugat congelé pour obtenir une poudre contenant de 90 à 98% de matière sèche.

La poudre obtenue par lyophilisation correspond à ce qu'on entend dans la présente demande par « forme sèche, de préférence lyophilisée ».

Selon un mode de réalisation de l'invention, la bactérie telle que ci-dessus définie, pour son utilisation dans la prévention et/ou le traitement des candidoses vaginales, se présente sous forme congelée ou sous forme sèche, de préférence sous forme sèche, et plus préférentiellement encore sous forme sèche lyophilisée.

Selon un mode de réalisation avantageux de l'invention, la souche de bactérie ou bactérie I-5777 est utilisée, pour son utilisation dans la prévention et/ou le traitement des candidoses vaginales, sous forme sèche, de préférence lyophilisée, à une dose quotidienne de 1x10⁷ à 1x10¹⁰ UFC, de préférence de 2x10⁸ à 2x10⁹ UFC.

Selon encore un autre mode de réalisation avantageux de l'invention, la souche de bactérie ou bactérie I-5778 est utilisée, sous forme sèche, de préférence lyophilisée, pour son utilisation dans la prévention et/ou le traitement des candidoses vaginales, à une dose quotidienne de 1x10⁷ à 1x10¹⁰ UFC, de préférence de 2x10⁸ à 2x10⁹ UFC.

L'acronyme « UFC » signifie « Unité Formant Colonie ».

Le terme « vaginal(e) » est utilisé dans la présente demande de manière large et les termes « vaginal(e) » et « vulvo-vaginal(e) » sont ici synonymes.

On entend par "candidose vaginale" un type particulier de mycose vaginale due à des champignons du genre *Candida,* comme par exemple ceux choisis dans le groupe comprenant *Candida albicans, Candida tropicalis, Candida pseudotropicalis, Candida krusei, Candida glabrata* et leurs mélanges.

La candidose vaginale est également appelée vaginite candidosique.

Selon un autre mode de réalisation, l'invention a pour objet une souche de *Limosilactobacillus fermentum* telle que ci-dessus définie ou une bactérie *Limosilactobacillus fermentum* telle que ci-dessus définie, pour son utilisation dans la prévention et/ou le traitement des candidoses vaginales dues à des champignons choisis dans le groupe comprenant *Candida albicans, Candida tropicalis, Candida pseudotropicalis, Candida krusei, Candida glabrata* et leurs mélanges.

La souche *Limosilactobacillus fermentum* ou la bactérie *Limosilactobacillus fermentum* peut être utilisée de manière préventive chez des femmes présentant des prédispositions et/ou une sensibilité aux candidoses vaginales, ou de manière curative, par exemple lors d'épisodes infectieux.

Les présents inventeurs ont également découvert que lorsque l'une de ces deux souches ou les deux souches de bactérie étaient associées avec la souche *Saccharomyces cerevisiae* déposée le 17 octobre 2007 auprès de la CNCM (Collection Nationale de Cultures de Microorganismes, 25, rue du Docteur Roux, 75724 Paris cedex 15, France) sous le numéro I-3856, alors on obtenait un effet synergique particulièrement surprenant conduisant à des moyens particulièrement efficaces pour traiter les candidoses vaginales.

A cet égard, un autre objet de l'invention réside dans une souche de bactérie ou bactérie *Limosilactobacillus fermentum* telle que définie ci-dessus, pour une utilisation dans la prévention et/ou le traitement des candidoses vaginales, caractérisée en ce qu'elle est utilisée en association avec la souche *Saccharomyces cerevisiae* déposée auprès de la CNCM le 17 octobre 2017 sous le numéro I-3856 et/ou avec la levure obtenue par culture de ladite souche *Saccharomyces cerevisiae.*

La souche de *Saccharomyces cerevisiae* est une souche de levure. L'expression « souche de levure » désigne une population relativement homogène de cellules de levure. Une souche de levure est obtenue à partir de l'isolement d'un clone, un clone étant une population de cellules obtenue à partir d'une seule cellule de levure.

La levure *Saccharomyces cerevisiae* désigne, au sens de la présente demande, la levure I-3856.

Ainsi, selon un mode de réalisation de l'invention, la souche de bactérie ou bactérie *Limosilactobacillus fermentum* peut être associée à la souche *Saccharomyces cerevisiae* et/ou à la levure *Saccharomyces cerevisiae.*

Autrement dit, selon un mode de réalisation avantageux de l'invention, la (les) bactérie(s) *Limosilactobacillus fermentum* I-5777 et/ou I-5778 est (sont) associée(s) à la levure *Saccharomyces cerevisiae* I-3856.

A titre indicatif, la levure *Saccharomyces cerevisiae* peut être obtenue par culture de la souche numéro I-3856 dans un milieu de culture, par exemple comme décrit dans le livre de référence « Yeast Technology », 2ème édition, 1991, G. Reed et T.W. Nagodawithana, publié par Van Nostrand Reinhold, ISBN 0-442-31892-8.

La levure Sc I-3856 peut cependant également être obtenue par un procédé à l'échelle industrielle qui comprend les étapes suivantes :
- culture d'une souche de levure dans un milieu de culture en plusieurs stades, d'abord en semi-anaérobiose, puis en aérobiose,
- séparation par centrifugation de la levure ainsi produite de son milieu de culture, pour obtenir une crème de levure liquide contenant de 12 à 25% de matière sèche, voire une quantité plus élevée de matière sèche, en particulier si la crème de levure est mélangée avec des produits osmolytes.

On utilisera indifféremment, dans la présente demande, les termes « levure obtenue par culture de la souche *Saccharomyces cerevisiae* déposée à la CNCM sous le numéro I-3856", "levure obtenue par culture de la souche I-3856" et "levure Sc I-3856", ces trois termes ayant la même signification telle que donnée ci-dessus.

La souche *Saccharomyces cerevisiae* déposée à la CNCM sous le numéro I-3856 peut encore être désignée par "souche Sc I-3856".

Selon encore un mode de réalisation avantageux de l'invention, la levure Sc I-3856, utilisée en association avec la souche de bactérie ou bactérie *Limosilactobacillus fermentum,* est vivante. La levure vivante se présente alors sous forme de levure sèche ou de levure fraîche, de préférence sous forme de levure sèche.

A titre d'exemple, dans le cas de l'association de la levure Sc I-3856 avec la souche bactérienne ou bactérie *Limosilactobacillus fermentum,* pour prévenir et/ou traiter les candidoses vaginales :
- la souche bactérienne ou bactérie *Limosilactobacillus fermentum,* sous forme sèche, de préférence lyophilisée, est utilisée à une dose quotidienne de 1x10⁷ à 1x10¹⁰ UFC, de préférence de 2x10⁸ à 2x10⁹ UFC et,
- la levure Sc I-3856, sous forme sèche, est utilisée à une dose quotidienne de 1x10⁷ à 1x10¹¹ UFC, de préférence de 1x10⁹ à 1x10¹⁰ UFC, et plus préférentiellement encore de 2,5x10⁹ à 5x10⁹ UFC.

Une levure fraîche est caractérisée par une teneur élevée en eau. Une levure fraîche est par exemple choisie parmi une levure liquide ou une levure pressée.

Une levure liquide, appelée également « crème de levure liquide », est une suspension aqueuse comprenant de 12% à 50% de matière sèche levure, plus généralement de 12% à 25% de matière sèche levure.

Une levure pressée est obtenue par a/ filtration d'une crème de levure liquide, en général sur un filtre rotatif sous vide, pour obtenir une levure fraîche déshydratée contenant de 26% à 37% de matière sèche, et par b/ malaxage de ladite levure fraîche déshydratée et c/ extrusion.

Une levure pressée comprend de 26% à 37% de matière sèche levure. La levure pressée peut être émiettée ou non.

Parmi les levures sèches, on peut par exemple citer la levure sèche active, la levure sèche instantanée et la levure à humidité intermédiaire surgelée.

Un avantage d'une levure sèche est sa longue durée de conservation.

Une levure sèche active ou une levure sèche instantanée comprend un taux de matière sèche levure supérieur à 90%, de préférence un taux de matière sèche compris de 92% à 96%.

La levure sèche active est obtenue par déshydratation de la levure pressée ou liquide par l'action conjointe de la chaleur (à basse température) et d'une activité mécanique, qui permettent de transformer un produit pâteux (levure pressée ou liquide) en un produit sec, sous forme de sphérules. Par exemple, la levure sèche active est obtenue par extrusion et séchage en lit fluidisé d'une levure pressée ou d'une levure liquide.

La levure sèche active se présente sous forme de sphérules dont le diamètre est généralement compris de 0,1 µm à 2,5 mm.

La levure sèche instantanée est obtenue par déshydratation de la levure pressée ou liquide par l'action d'un gradient d'air chaud qui permet de transformer un produit pâteux (levure pressée ou liquide) en de fins vermicelles secs. La levure sèche instantanée doit ensuite, pour rester stable, être conditionnée en absence d'oxygène.

La levure sèche à humidité intermédiaire surgelée comprend par exemple de 70% à 85% de matière sèche levure.

Selon encore un mode de réalisation avantageux de l'invention, la levure Sc I-3856, utilisée en association avec la souche de bactérie ou bactérie *Limosilactobacillus fermentum,* dans la prévention et/ou le traitement des mycoses vaginales, se présente sous forme de levure sèche active ou sous forme de levure sèche instantanée.

Selon encore un mode de réalisation avantageux de l'invention, la levure Sc I-3856, utilisée en association avec la souche de bactérie ou bactérie *Limosilactobacillus fermentum,* dans la prévention et/ou le traitement des mycoses vaginales, se présente sous forme de levure morte, appelée également levure désactivée.

Une levure morte est une levure dont le métabolisme est irrémédiablement arrêté.

Une levure morte peut être obtenue par des techniques bien connues de l'homme du métier, telles qu'un traitement thermique de la levure, un traitement consistant à soumettre la levure à plusieurs cycles de congélation et décongélation successives, un traitement par irradiation, un traitement par atomisation ou une combinaison de ces traitements.

A titre d'exemple, dans le cas de l'association de la levure morte avec la souche bactérienne ou bactérie *Limosilactobacillus fermentum,* pour prévenir et/ou traiter les candidoses vaginales :
- la souche bactérienne ou bactérie *Limosilactobacillus fermentum,* sous forme sèche, de préférence lyophilisée, est utilisée à une dose quotidienne de 1x10⁷ à 1x10¹⁰ UFC, de préférence de 2x10⁸ à 2x10⁹ UFC et,
- la levure Sc I-3856, sous forme de levure morte, est utilisée à une dose quotidienne de 1 à 10 000 mg, et de préférence de 100 à 1000 mg.

Selon encore un mode de réalisation avantageux de l'invention, la levure Sc I-3856, utilisée en association avec la souche de bactérie ou bactérie *Limosilactobacillus fermentum,* est utilisée sous forme de dérivé de levure, ledit dérivé étant choisi parmi la paroi de ladite levure, les glucanes pariétaux de ladite levure, les mannoprotéines pariétales de ladite levure ou leurs mélanges.

La paroi de levure désigne de manière large à la fois la paroi et la membrane plasmique de la levure. De façon classique, la paroi de levure est obtenue par un procédé comprenant une étape d'autolyse de la levure suivie d'une étape de séparation de la fraction soluble de la fraction insoluble, la fraction insoluble isolée correspondant à la paroi de levure qui peut ensuite être séchée.

A titre d'exemple, dans le cas de l'association d'une paroi de levure Sc I-3856 avec la souche bactérienne ou bactérie *Limosilactobacillus fermentum,* pour prévenir et/ou traiter les candidoses vaginales :
- la souche bactérienne ou bactérie *Limosilactobacillus fermentum,* sous forme sèche, de préférence lyophilisée, est utilisée à une dose quotidienne de 1x10⁷ à 1x10¹⁰ UFC, de préférence de 2x10⁸ à 2x10⁹ UFC et,
- la paroi de levure Sc I-3856 est utilisée à une dose quotidienne de 0,5 à 5 000 mg, de préférence de 50 à 500 mg.

A titre d'exemple, dans le cas de l'association de glucanes pariétaux de levure Sc I-3856 avec la souche bactérienne ou bactérie *Limosilactobacillus fermentum,* pour prévenir et/ou traiter les candidoses vaginales :
- la souche bactérienne ou bactérie *Limosilactobacillus fermentum,* sous forme sèche, de préférence lyophilisée, est utilisée à une dose quotidienne de 1x10⁷ à 1x10¹⁰ UFC, de préférence de 2x10⁸ à 2x10⁹ UFC et,
- les glucanes pariétaux de levure Sc I-3856 sont utilisés à une dose quotidienne de 0,125 à 1250 mg, et de préférence de 12,5 à 125 mg.

Toujours à titre d'exemple, dans le cas de l'association de mannoprotéines pariétales de levure Sc I-3856 avec la souche bactérienne ou bactérie *Limosilactobacillus fermentum,* pour prévenir et/ou traiter les candidoses vaginales :
- la souche bactérienne ou bactérie *Limosilactobacillus fermentum,* sous forme sèche, de préférence lyophilisée, est utilisée à une dose quotidienne de 1x10⁷ à 1x10¹⁰ UFC, de préférence de 2x10⁸ à 2x10⁹ UFC et,
- les mannoprotéines pariétales de levure Sc I-3856 sont utilisées à une dose quotidienne de 0,125 à 1250 mg, et de préférence de 12,5 à 125 mg.

Selon un mode de réalisation avantageux de l'invention, la souche bactérienne et/ou bactérie *Limosilactobacillus fermentum* d'une part et, la souche Sc I-3856 et/ou levure Sc I-3856 d'autre part, sont administrées de façon simultanée ou séquentielle, et de préférence simultanée.

Lorsque l'administration est séquentielle, la levure Sc I-3856 pourra par exemple être administrée le matin et la (les) bactérie(s) I-5777 et/ou I-5778 le soir.

Ainsi, l'invention concerne encore la souche bactérienne et/ou bactérie *Limosilactobacillus fermentum,* en association avec la souche Sc I-3856 et/ou levure Sc I-3856, pour une administration simultanée ou séquentielle, pour une utilisation dans la prévention et/ou le traitement des candidoses vaginales.

En fonction du mode d'administration recherché (simultané ou séquentiel), la souche bactérienne et/ou bactérie *Limosilactobacillus fermentum* d'une part, et la souche Sc I-3856 et/ou levure Sc I-3856 d'autre part, se trouvent sous la forme d'une composition unique ou sous la forme d'une composition séparée.

Une composition unique signifie que la souche bactérienne et/ou bactérie *Limosilactobacillus fermentum* d'une part et la souche Sc I-3856 et/ou levure Sc I-3856 d'autre part se trouvent au sein d'une seule et même composition.

Une composition « séparée » signifie que la souche bactérienne et/ou bactérie *Limosilactobacillus fermentum* se trouve(nt) dans une composition et la souche Sc I-3856 et/ou levure Sc I-3856 se trouve(nt) dans une autre composition, distincte de la composition comprenant la souche bactérienne et/ou bactérie.

Selon un mode de réalisation préféré, l'invention porte sur la souche bactérienne et/ou bactérie I-5777, en association avec la souche Sc I-3856 et/ou levure Sc I-3856, pour une utilisation dans la prévention et/ou le traitement des candidoses vaginales.

De préférence la souche bactérienne et/ou bactérie I-5777 d'une part, et la souche Sc I-3856 et/ou levure Sc I-3856 d'autre part, sont présentes au sein d'une composition unique et sont donc administrées simultanément.

Selon un autre mode de réalisation préféré, l'invention porte sur la souche bactérienne et/ou bactérie I-5778, en association avec la souche Sc I-3856 et/ou levure Sc I-3856, pour une utilisation dans la prévention et/ou le traitement des candidoses vaginales. De préférence la souche bactérienne et/ou bactérie I-5778 d'une part et la souche Sc I-3856 et/ou levure Sc I-3856 d'autre part, sont présentes au sein d'une composition unique et sont donc administrées simultanément.

Encore un autre objet de l'invention porte sur une composition comprenant :
- une souche *Limosilactobacillus fermentum* choisie parmi la souche déposée auprès de la CNCM le 17 novembre 2021 sous le numéro I-5777 ou sous le numéro I-5778, et/ou une bactérie obtenue par culture de ladite souche *Limosilactobacillus fermentum,*
- éventuellement un excipient,
pour son utilisation dans la prévention et/ou le traitement des candidoses vaginales.

Un autre objet de l'invention réside dans une composition comprenant :
- une souche *Limosilactobacillus fermentum* choisie parmi la souche déposée auprès de la CNCM le 17 novembre 2021 sous le numéro I-5777 ou sous le numéro I-5778, et/ou une bactérie obtenue par culture de ladite souche *Limosilactobacillus fermentum,*
- la souche *Saccharomyces cerevisiae* déposée auprès de la CNCM le 17 octobre 2007 sous le numéro I-3856 et/ou la levure obtenue par culture de ladite souche *Saccharomyces cerevisiae,*
- éventuellement un excipient,
pour son utilisation dans la prévention et/ou le traitement des candidoses vaginales.

La composition selon l'invention pour une utilisation telle que ci-dessus définie peut être une composition pharmaceutique, un complément alimentaire ou une composition alimentaire.

La composition alimentaire désigne n'importe quel aliment, boisson ou confiserie. A titre d'exemple de composition alimentaire on pourra citer une barre de céréales, un chewing-gum, un produit laitier.

Lorsque la composition est une composition alimentaire alors des exemples d'excipient sont le lactose, l'amidon de maïs, le carboxyméthylcellulose, le mannitol, le sucrose.

On entend par « complément alimentaire » une denrée alimentaire dont le but est de compléter le régime alimentaire normal et qui constitue une source concentrée de nutriments ou d'autres substances ayant un effet nutritionnel ou physiologique, seuls ou combinés.

Un complément alimentaire est commercialisé sous forme de dose, par exemple les formes de présentation telles que gélule, pastille, comprimé, pilule et autres formes similaires, sachet de poudre, ampoule de liquide, flacon muni d'un compte-goutte et autres formes analogues de préparations liquides ou en poudres destinées à être prises en unités mesurées de faible quantité.

La composition alimentaire et le complément alimentaire sont destinés à une administration par voie orale.

La composition pharmaceutique de l'invention est destinée à une administration par voie orale ou vaginale, de préférence par voie orale. Les excipients mis en œuvre dans la composition pharmaceutique sont des excipients classiquement utilisés et qui sont adaptés à la préparation de formes orales ou vaginales.

A titre d'exemple de composition pharmaceutique se présentant sous une forme adaptée pour la voie orale on peut citer un comprimé, une capsule, une gélule, un sachet, une poudre, une crème, un sirop ou une ampoule.

A titre d'exemple de composition pharmaceutique se présentant sous une forme adaptée pour la voie vaginale on peut citer un ovule, une capsule, une gélule, une crème ou un comprimé.

Le dosage journalier dépend à la fois du mode d'administration (voie orale ou voie vaginale) et du type de traitement (curatif ou préventif).

La composition pour une utilisation telle que ci-dessus définie se trouve sous une forme adaptée pour une administration par voie orale ou vaginale, de préférence par voie orale.

Selon un mode de réalisation avantageux de l'invention, la composition pour une utilisation telle que ci-dessus définie ne comprend pas d'autres ingrédients actifs que la souche *Limosilactobacillus fermentum* I-5777 et/ou I-5778 et/ou la bactérie *Limosilactobacillus fermentum* I-5777 et/ou I-5778 d'une part, et la souche Sc I-3856 et/ou la levure Sc I-3856 d'autre part.

Ainsi, la présente invention porte encore sur une composition pour une utilisation telle que ci-dessus définie, caractérisée en ce qu'elle est comprend ou en ce qu'elle est constituée :
- de la souche *Limosilactobacillus fermentum* I-5777 et/ou I-5778 et/ou de la bactérie *Limosilactobacillus fermentum* I-5777 et/ou I-5778 d'une part,
- de la souche Sc I-3856 et/ou de la levure Sc I-3856 d'autre part,
- éventuellement d'un excipient.

Si la composition comprend un excipient, alors l'excipient sera choisi en fonction de la destination de la composition (pharmaceutique, alimentaire, complément alimentaire).

Selon un autre mode de réalisation avantageux de l'invention, la composition pour une utilisation telle que ci-dessus définie est caractérisée en ce qu'elle comprend en outre un ingrédient actif, notamment choisi dans le groupe comprenant la vitamine K, la vitamine B9, le glutathion, la S-adénosyl-L-méthionine (SAM-e), le sulfate de chondroïtine et leurs mélanges. Une telle composition est de préférence destinée à une administration par voie orale.

Selon encore un autre mode de réalisation de l'invention, la composition, pour une utilisation telle que ci-dessus définie, est plus particulièrement caractérisée en ce que :
- la souche bactérienne ou bactérie *Limosilactobacillus fermentum* I-5777 et/ou I-5778 est sous forme sèche, de préférence lyophilisée, et est présente en une quantité allant de 1x10⁷ à 1x10¹⁰ UFC, de préférence de 2x10⁸ à 2x10⁹ UFC et,
- la levure Sc I-3856 est sous forme sèche, et est présente en une quantité allant de 1x10⁷ à 1x10¹¹ UFC, de préférence de 1x10⁹ à 1x10¹⁰ UFC, et plus préférentiellement encore de 2,5x10⁹ à 5x10⁹ UFC.

Selon un autre mode de réalisation, la composition, pour une utilisation telle que ci-dessus définie, est plus particulièrement caractérisée en ce que :
- la souche bactérienne ou bactérie *Limosilactobacillus fermentum* I-5777 et/ou I-5778 est sous forme sèche, de préférence lyophilisée, et est présente en une quantité allant de 1x10⁷ à 1x10¹⁰ UFC, de préférence de 2x10⁸ à 2x10⁹ UFC et,
- la levure Sc I-3856 est sous forme de levure morte, et est présente en une quantité allant de 1 à 10 000 mg, et de préférence de 100 à 1000 mg.

Selon encore un autre mode de réalisation, la composition, pour une utilisation telle que ci-dessus définie, est plus particulièrement caractérisée en ce que :
- la souche bactérienne ou bactérie *Limosilactobacillus fermentum* I-5777 et/ou I-5778 est sous forme sèche, de préférence lyophilisée, et est présente en une quantité allant de 1x10⁷ à 1x10¹⁰ UFC, de préférence de 2x10⁸ à 2x10⁹ UFC et,
- la levure Sc I-3856 est sous forme de paroi de levure, et est présente en une quantité allant de 0,5 à 5 000 mg, de préférence de 50 à 500 mg.

Selon un autre mode de réalisation, la composition, pour une utilisation telle que ci-dessus définie, est plus particulièrement caractérisée en ce que :
- la souche bactérienne ou bactérie *Limosilactobacillus fermentum* I-5777 et/ou I-5778 est sous forme sèche, de préférence lyophilisée, et est présente en une quantité allant de 1x10⁷ à 1x10¹⁰ UFC, de préférence de 2x10⁸ à 2x10⁹ UFC et,
- la levure Sc I-3856 est sous forme de glucanes pariétaux et est présente en une quantité allant de 0,125 à 1250 mg, de préférence de 12,5 à 125 mg.

Selon un autre mode de réalisation, la composition, pour une utilisation telle que ci-dessus définie, est plus particulièrement caractérisée en ce que :
- la souche bactérienne ou bactérie *Limosilactobacillus fermentum* I-5777 et/ou I-5778 est sous forme sèche, de préférence lyophilisée, et est présente en une quantité allant de 1x10⁷ à 1x10¹⁰ UFC, de préférence de 2x10⁸ à 2x10⁹ UFC et,
- la levure Sc I-3856 est sous forme de mannoprotéines pariétales et est présente en une quantité allant de 0,125 à 1250 mg, de préférence de 12,5 à 125 mg.

### Brève description des dessins

D'autres caractéristiques, détails et avantages apparaîtront à la lecture de la description détaillée ci-après, et à l'analyse des dessins annexés.
**Fig. 1**

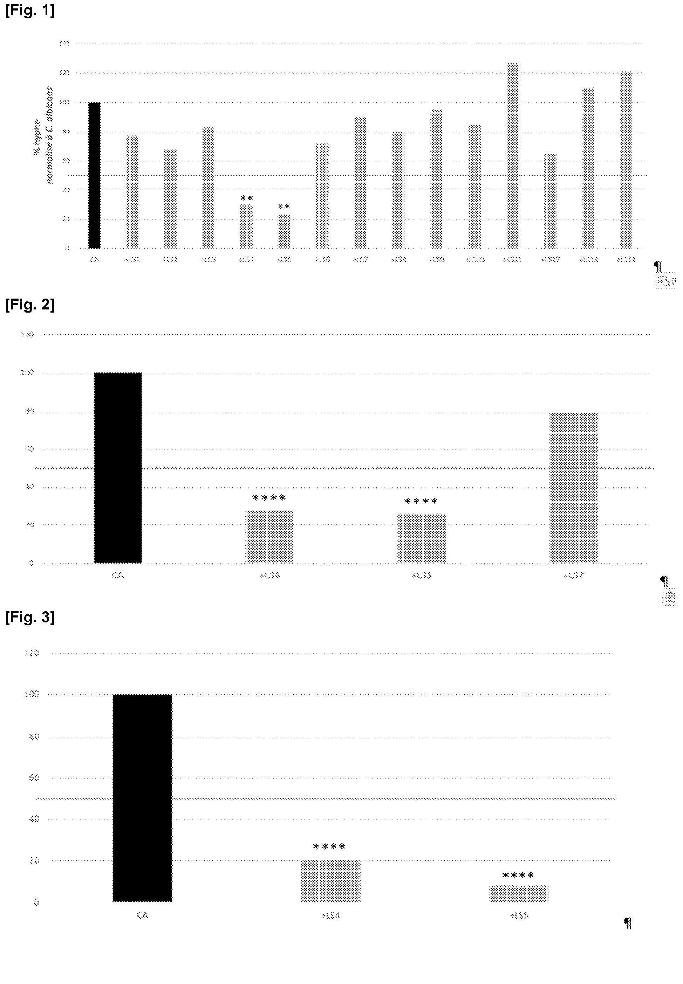

   [Fig. 1] est un histogramme résultant d'une première expérimentation et qui montre le pourcentage d'inhibition de la formation des hyphes de *Candida albicans* par des lactobacilles dénommées « LS1 » à « LS14 », les lactobacilles LS4 et LS5 correspondants aux souches bactériennes de l'invention, à savoir respectivement les souches CNCM I-5777 et CNCM I-5778.
   L'axe des ordonnées représente le pourcentage de formation d'hyphe normalisé à *Candida albicans.*
   Le groupe « CA » (*Candida albicans*) représente le groupe témoin.
   Les différences significatives comparées au groupe témoin sont annotées avec astérisque ** p<0,01.
**Fig. 2**
   [Fig. 2] est un histogramme résultant d'une deuxième expérimentation et qui montre le pourcentage d'inhibition de la formation des hyphes de *Candida albicans* par respectivement le témoin CA et les lactobacilles LS4 (CNCM I-5777), LS5 (CNCM I-5778) et LS7.
   L'axe des ordonnées représente le pourcentage de formation d'hyphe normalisé à *Candida albicans.*
   Les différences significatives comparées au groupe témoin sont annotées avec astérisque **** p<0,0001.
**Fig. 3**
   [Fig. 3] est un histogramme résultant d'une troisième expérimentation et qui montre le pourcentage d'inhibition de la formation des hyphes de *Candida albicans* par respectivement le témoin CA et les lactobacilles LS4 (CNCM I-5777) et LS5 (CNCM I-5778). L'axe des ordonnées représente le pourcentage de formation d'hyphe normalisé à *Candida albicans.*
   Les différences significatives comparées au groupe témoin sont annotées avec astérisque **** p<0,0001.
**Fig. 4**

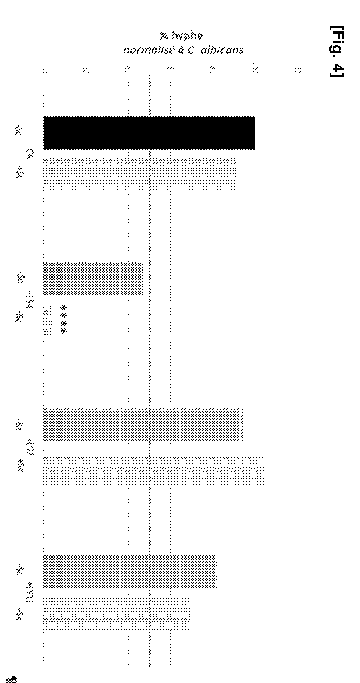

   [Fig. 4] est un histogramme qui montre le pourcentage d'inhibition de la formation des hyphes de *Candida albicans* par respectivement le témoin CA, seul ou combiné avec *Saccharomyces cerevisiae* CNCM I-3856 (« Sc »), et les lactobacilles (LS4, LS7 et LS11) seuls (histogrammes gris clairs) ou combinés avec *Saccharomyces cerevisiae* CNCM I-3856 (Sc) (histogrammes hachurés).
   L'axe des ordonnées représente le pourcentage de formation d'hyphe normalisé à *Candida albicans.*
   « -Sc » signifie l'absence de *Saccharomyces cerevisiae* tandis que « +Sc » signifie la présence de *Saccharomyces cerevisiae.*
   Les différences significatives comparées au groupe témoin sont annotées avec astérisque **** p<0,0001.

### Description des modes de réalisation

Dans l'exemple 1 ci-dessous il est fait référence aux Figures 1 à 4.

### Exemple 1

### Test des hyphes

Le test des hyphes, réalisé en conditions *in vitro,* permet d'évaluer la capacité de souches (de bactérie ou de levure) à bloquer la formation des hyphes (c'est-à-dire à inhiber la formation des hyphes) de *Candida albicans.*

En effet, la formation des hyphes est une étape cruciale dans la virulence et la capacité d'invasion de *Candida albicans* : la morphogenèse hyphale est ainsi un important facteur de virulence de C. *albicans.*

L'inhibition/réduction des hyphes représente une approche intéressante pour combattre les infections à *Candida.*

### Souches testées

Quatorze souches de lactobacilles ont été testées (LS1 à LS14), seules ou en association avec la souche de S. *cerevisiae* CNCM I-3856.

Les souches LS1 à LS14 sont des souches isolées à partir de levain.
LS1 : *Lactiplantibacillus plantarum,* référence d'identification BCC_002300,
LS2 : *Lactiplantibacillus plantarum,* référence d'identification BCC_002377,
LS3 : *Lactiplantibacillus plantarum,* référence d'identification BCC_003240,
LS4 : *Limosilactobacillus fermentum,* référence d'identification BCC_002284 (souche déposée auprès de la CNCM le 17 novembre 2021 sous le numéro I-5777),
LS5 : *Limosilactobacillus fermentum,* référence d'identification BCC_002321 (souche déposée auprès de la CNCM le 17 novembre 2021 sous le numéro I-5778),
LS6 : *Lacticaseibacillus* paracasei, référence d'identification BCC_002365,
LS7 : *Lactiplantibacillus plantarum,* référence d'identification BCC_002378 (souche déposée auprès de la CNCM le 17 novembre 2021 sous le numéro I-5779),
LS8 : *Lactiplantibacillus plantarum,* référence d'identification BCC_004104,
LS9 : *Lactiplantibacillus plantarum,* référence d'identification BCC_004132,
LS10 : *Lactiplantibacillus plantarum,* référence d'identification BCC_004299,
LS11 : *Lacticaseibacillus paracasei*, référence d'identification BCC_002274,
LS12 : *Lacticaseibacillus paracasei,* référence d'identification BCC_002301,
LS13 : *Lacticaseibacillus paracasei,* référence d'identification BCC_003027,
LS14 : *Lacticaseibacillus paracasei,* référence d'identification BCC_003258.

### Protocole des tests

### Protocole avec les lactobacilles seuls

Les concentrations de cultures d'une nuit des souches de lactobacilles (LS1 à LS14) et des souches de *C. albicans* sont estimées par spectrophotométrie (densité optique à 600 nm). Les cultures sont ensuite ajustées à une concentration de 1x10⁹ UFC/ml pour les lactobacilles et de 1x10*⁶* CFU/ml pour *C. albicans.*

Des mélanges de *C. albicans* avec les lactobacilles sont réalisés en ajoutant 50 µl de suspension de chaque microorganisme testé à 125 µl de sérum de veau fœtal (FCS), additionné de bouillon YPD (bouillon d'extrait de levure peptone-dextrose) pour un volume total de 500 µl.

Pour chaque condition, quatre répétitions techniques sont incluses (les résultats proviennent de 4 puits différents mais avec une même culture d'une nuit au départ). Après 3 heures d'incubation à 37°C, 2 µl des mélanges sont utilisés pour une évaluation microscopique en comptant le nombre de cellules de levure et le nombre de cellules formant des hyphes. Au moins 100 cellules sont comptées et les rapports sont calculés et normalisés par rapport au contrôle négatif (c'est-à-dire *C. albicans +* FCS).

### Protocole avec les lactobacilles en combinaison avec Saccharomyces cerevisiae

Le surnageant de culture d'une nuit de *S. cerevisiae* (Sc I-3856) est obtenu par centrifugation (10 min, 2000 g) et filtration (0,20µm filtre d'acétate de cellulose).

Les concentrations de cultures d'une nuit des souches de lactobacilles et *C. albicans* sont estimées par spectrophotométrie (densité optique à 600 nm). Les cultures sont ensuite ajustées à une concentration de 1x10⁹ UFC/ml pour les lactobacilles et 1x10⁶ CFU/ml pour *C. albicans.*

Des mélanges de *C. albicans* avec des lactobacilles et le surnageant de *S. cerevisiae* sont réalisés en ajoutant 50 µl de suspension de chaque microorganisme testé à 125 µl de sérum de veau fœtal (FCS), additionné de bouillon YPD pour un volume total de 500 µl.

La suite du protocole est identique au protocole décrit précédemment pour les lactobacilles seuls.

### Résultats

Les résultats obtenus sont commentés ci-après et sont illustrés par les Figures 1 à 4. On note, dans chacune de ces figures, zéro pour cent (0 %) d'inhibition de la formation des hyphes avec le témoin CA (*C. albicans*) (ce qui signifie 100% de formation des hyphes de *C. albicans*).

### Lactobacilles seuls (Figures 1 à 3)

Il ressort de la Figure 1 que, parmi les 14 lactobacilles testés (LS1 à LS14), seules les souches *Limosilactobacillus fermentum* LS4 (CNCM I-5777) et LS5 (CNCM I-5778) permettent une forte inhibition de la formation des hyphes de *C. albicans.*

En effet, avec les souches LS4 et LS5 on observe respectivement environ 72% d'inhibition de la formation des hyphes (i.e. environ 28% de formation des hyphes) (LS4) et environ 78% d'inhibition de la formation des hyphes de *C. albicans* (i.e. environ 22% de formation des hyphes) (LS5).

Dans la Figure 2, les souches LS4 (CNCM I-5777) et LS5 (CNCM I-5778) ont été comparées à la souche LS7 (CNCM I-5779) car cette souche est représentative d'un grand nombre de souches testées.

Il ressort également de la Figure 2 que les souches LS4 (CNCM I-5777) et LS5 (CNCM I-5778) permettent une forte inhibition de la formation des hyphes de *C. albicans* en comparaison de la souche LS7 (CNCM I-5779). En effet, avec les souches LS4 et LS5 on observe respectivement environ 74% d'inhibition (LS4) et environ 75% d'inhibition (LS5) tandis qu'avec la souche LS7 (CNCM I-5779) l'inhibition est à peine de 20%.

Dans la Figure 3 seules les souches LS4 (CNCM I-5777) et LS5 (CNCM I-5778) ont été comparées. A nouveau, les résultats obtenus sont prometteurs puisque ces souches conduisent à une forte inhibition de la formation des hyphes de *C. albicans,* à savoir respectivement environ 81% (LS4) et 93% (LS5) d'inhibition de la formation des hyphes de *C. albicans.*

### Lactobacilles en combinaison avec Saccharomyces cerevisiae (Figure 4)

Il ressort de la Figure 4 que l'association de LS4 (CNCM I-5777) avec *S. cerevisiae* CNCM I-3856 conduit à un effet synergique inattendu, ce qui n'est pas le cas de l'association respective des souches LS7 (CNCM I-5779) et LS11 avec Sc I-3856.

En effet, l'utilisation de la souche *S. cerevisiae* CNCM I-3856 (voir « CA +Sc ») seule (c'est-à-dire non associée à un lactobacille) conduit à une inhibition de la formation des hyphes de *C. albicans* d'environ 9% dans les conditions expérimentales mises en jeu. L'utilisation de la souche LS4 seule (voir « LS4 -Sc ») conduit à une inhibition de la formation des hyphes de *C. albicans* d'environ 52%. Cependant, lorsque les souches LS4 et Sc CNCM I-3856 sont associées, alors l'inhibition de la formation des hyphes de *C. albicans* est presque totale puisqu'elle est d'environ 97%.

La présente invention ne se limite pas aux exemples décrits ci-avant, seulement à titre d'exemple, mais elle englobe toutes les variantes que pourra envisager l'homme de l'art dans le cadre de la protection recherchée. telle que définie dans les revendications.

### Référence à du matériel biologique déposé

Dans la présente demande, il est fait référence aux matériels biologiques suivants et aux souches variantes ou mutantes dérivées :
- référence d'identification « BCC_002284 » et numéro d'enregistrement « I-5777 » déposée à la Collection Nationale de Cultures de Micro-organismes (CNCM) (25, rue du Docteur Roux, 75724 Paris cedex 15) en France, le 17 novembre 2021 par Lesaffre et Compagnie dont l'adresse est 41 rue Etienne Marcel, 75009 Paris ;
- référence d'identification « BCC_002321 » et numéro d'enregistrement « I-5778 » déposée à la Collection Nationale de Cultures de Micro-organismes (CNCM) (25, rue du Docteur Roux, 75724 Paris cedex 15) en France, le 17 novembre 2021 par Lesaffre et Compagnie dont l'adresse est 41 rue Etienne Marcel, 75009 Paris ;
- référence d'identification « BCC_002378 » et numéro d'enregistrement « I-5779 » déposée à la Collection Nationale de Cultures de Micro-organismes (CNCM) (25, rue du Docteur Roux, 75724 Paris cedex 15) en France, le 17 novembre 2021 par Lesaffre et Compagnie dont l'adresse est 41 rue Etienne Marcel, 75009 Paris ;
- référence d'identification « SCPro-1 » et numéro d'enregistrement « I-3856 » déposée à la Collection Nationale de Cultures de Micro-organismes (CNCM) (25, rue du Docteur Roux, 75724 Paris cedex 15) en France, le 17 octobre 2007 par Lesaffre et Compagnie dont l'adresse est 41 rue Etienne Marcel, 75009 PARIS.

## Revendications

1. Souche *Limosilactobacillus fermentum* choisie parmi la souche déposée auprès de la CNCM le 17 novembre 2021 sous le numéro I-5777 ou sous le numéro I-5778, pour son utilisation dans la prévention et/ou le traitement des candidoses vaginales.

2. Bactérie obtenue par culture de la souche *Limosilactobacillus fermentum* choisie parmi la souche déposée auprès de la CNCM le 17 novembre 2021 sous le numéro I-5777 ou sous le numéro I-5778, pour son utilisation dans la prévention et/ou le traitement des candidoses vaginales.

3. Bactérie pour une utilisation selon la revendication 2, **caractérisée en ce qu'**elle se présente sous forme congelée ou sous forme sèche, de préférence sous forme sèche, et plus préférentiellement encore sous forme sèche lyophilisée.

4. Souche pour une utilisation selon la revendication 1 ou bactérie pour une utilisation selon la revendication 2 ou 3, **caractérisée en ce que** les candidoses vaginales sont dues à des champignons choisis dans le groupe comprenant *Candida albicans, Candida tropicalis, Candida pseudotropicalis, Candida krusei, Candida glabrata* et leurs mélanges.

5. Souche ou bactérie pour une utilisation selon l'une quelconque des revendications 1 à 4, **caractérisée en ce qu'**elle est utilisée en association avec la souche *Saccharomyces cerevisiae* déposée auprès de la CNCM le 17 octobre 2007 sous le numéro I-3856 et/ou avec la levure obtenue par culture de ladite souche *Saccharomyces cerevisiae.*

6. Souche ou bactérie pour une utilisation selon la revendication 5, **caractérisée en ce que** la levure obtenue par culture de la souche *Saccharomyces cerevisiae* se présente sous forme de levure sèche ou de levure fraîche, et de préférence sous forme de levure sèche.

7. Souche ou bactérie pour une utilisation selon la revendication 6, **caractérisée en ce que** la levure sèche se présente sous forme de levure sèche active ou de levure sèche instantanée.

8. Souche ou bactérie pour une utilisation selon la revendication 5, **caractérisée en ce que** la levure se présente sous forme de levure morte.

9. Souche ou bactérie pour une utilisation selon la revendication 5, **caractérisée en ce que** la levure se présente sous forme de dérivé de levure, ledit dérivé étant choisi parmi la paroi de ladite levure, les glucanes pariétaux de ladite levure, les mannoprotéines pariétales de ladite levure ou leurs mélanges.

10. Composition comprenant :
- une souche *Limosilactobacillus fermentum* choisie parmi la souche déposée auprès de la CNCM le 17 novembre 2021 sous le numéro I-5777 ou sous le numéro I-5778, et/ou une bactérie obtenue par culture de ladite souche *Limosilactobacillus fermentum,*
- la souche *Saccharomyces cerevisiae* déposée auprès de la CNCM le 17 octobre 2007 sous le numéro I-3856 et/ou la levure obtenue par culture de ladite souche *Saccharomyces cerevisiae,*
- éventuellement un excipient,
pour son utilisation dans la prévention et/ou le traitement des candidoses vaginales.

11. Composition pour une utilisation selon la revendication 10, **caractérisée en ce que** la composition est une composition pharmaceutique, une composition alimentaire ou un complément alimentaire.

12. Composition pour une utilisation selon la revendication 10 ou la revendication 11, **caractérisée en ce qu'**elle se trouve sous une forme adaptée à une administration par voie orale ou par voie vaginale, de préférence par voie orale.

13. Composition pour une utilisation selon l'une quelconque des revendications 10 à 12, **caractérisée en ce qu'**elle comprend en outre un ingrédient actif choisi dans le groupe comprenant la vitamine K, la vitamine B9, le glutathion, la S-adénosyl-L-méthionine (SAM-e), le sulfate de chondroïtine et leurs mélanges.

14. Composition pour une utilisation selon l'une quelconque des revendications 10 à 13, dans laquelle :
- la souche bactérienne ou bactérie *Limosilactobacillus fermentum* I-5777 et/ou I-5778 est sous forme sèche, de préférence lyophilisée, et est présente en une quantité allant de 1x10⁷ à 1x10¹⁰ UFC, de préférence de 2x10⁸ à 2x10⁹ UFC et,
- la levure Sc I-3856 est sous forme sèche, et est présente en une quantité allant de 1x10⁷ à 1x10¹¹ UFC, de préférence de 1x10⁹ à 1x10¹⁰ UFC, et plus préférentiellement encore de 2,5x10⁹ à 5x10⁹ UFC.

## Patentansprüche

1. Stamm *Limosilactobacillus fermentum,* gewählt aus dem am 17. November 2021 bei der CNCM unter der Nummer I-5777 oder unter der Nummer I-5778 hinterlegten Stamm, zur Verwendung bei der Vorbeugung und/oder Behandlung von vaginalen Candidosen.

2. Bakterium, erhalten durch Kultivierung des Stammes *Limosilactobacillus fermentum,* gewählt aus dem am 17. November 2021 bei der CNCM unter der Nummer I-5777 oder unter der Nummer I-5778 hinterlegten Stamm, zur Verwendung bei der Vorbeugung und/oder Behandlung von vaginalen Candidosen.

3. Bakterium zur Verwendung nach Anspruch 2, **dadurch gekennzeichnet, dass** es in gefrorener oder getrockneter Form, bevorzugt in getrockneter Form und stärker bevorzugt in lyophilisierter Form vorliegt.

4. Stamm zur Verwendung nach Anspruch 1 oder Bakterium zur Verwendung nach Anspruch 2 oder 3, **dadurch gekennzeichnet, dass** die vaginale Candidosen durch Pilze verursacht wird, die gewählt sind aus der Gruppe, umfassend *Candida albicans, Candida tropicalis, Candida pseudotropicalis, Candida krusei, Candida glabrata* und deren Mischungen.

5. Stamm oder Bakterium zur Verwendung nach einem der Ansprüche 1 bis 4, **dadurch gekennzeichnet, dass** er in Verbindung mit dem Stamm *Saccharomyces cerevisiae,* der am 17. Oktober 2007 bei der CNCM unter der Nummer I-3856 hinterlegt wurde, und/oder mit der durch Kultivierung des genannten Stammes *Saccharomyces cerevisiae* erhaltenen Hefe verwendet wird.

6. Stamm oder Bakterium zur Verwendung nach Anspruch 5, **dadurch gekennzeichnet, dass** die durch Kultivierung des Stammes *Saccharomyces cerevisiae* gewonnene Hefe in Form von Trockenhefe oder Frischhefe und bevorzugt in Form von Trockenhefe vorliegt.

7. Stamm oder Bakterium zur Verwendung nach Anspruch 6, **dadurch gekennzeichnet, dass** die Trockenhefe in Form von aktiver Trockenhefe oder Instant-Trockenhefe vorliegt.

8. Stamm oder Bakterium zur Verwendung nach Anspruch 5, **dadurch gekennzeichnet, dass** die Hefe in Form von toter Hefe vorliegt.

9. Stamm oder Bakterium zur Verwendung nach Anspruch 5, **dadurch gekennzeichnet, dass** die Hefe in Form eines Hefederivats vorliegt, wobei das Derivat aus der Wand der Hefe, den Parietalglucanen der Hefe, den Parietalmannoproteinen der Hefe oder deren Mischungen gewählt ist.

10. Zusammensetzung, umfassend:
- einen Stamm *Limosilactobacillus fermentum,* gewählt aus dem Stamm, der am 17. November 2021 bei der CNCM unter der Nummer I-5777 oder unter der Nummer I-5778 hinterlegt wurde, und/oder ein Bakterium, das durch Kultivierung des genannten Stamms *Limosilactobacillus fermentum* erhalten wurde,
- den Stamm *Saccharomyces cerevisiae,* der bei der CNCM am 17. Oktober 2007 unter der Nummer I-3856 hinterlegt wurde, und/oder die durch Kultivierung des Stamms *Saccharomyces cerevisiae* erhaltene Hefe,
- gegebenenfalls einen Hilfsstoff,
zur Verwendung bei der Vorbeugung und/oder Behandlung von vaginalen Candidosen.

11. Zusammensetzung zur Verwendung nach Anspruch 10, **dadurch gekennzeichnet, dass** die Zusammensetzung eine pharmazeutische Zusammensetzung, eine Nahrungszusatzzusammensetzung oder ein Nahrungsergänzungsmittel ist.

12. Zusammensetzung zur Verwendung nach Anspruch 10 oder Anspruch 11, **dadurch gekennzeichnet, dass** sie in einer Form vorliegt, die zur oralen oder vaginalen Verabreichung, bevorzugt zur oralen Verabreichung, geeignet ist.

13. Zusammensetzung zur Verwendung nach einem der Ansprüche 10 bis 12, **dadurch gekennzeichnet, dass** sie ferner einen Wirkstoff umfasst, der aus der Gruppe gewählt ist, umfassend Vitamin K, Vitamin B9, Glutathion, S-Adenosyl-L-Methionin (SAM-e), Chondroitinsulfat und deren Mischungen.

14. Zusammensetzung zur Verwendung nach einem der Ansprüche 10 bis 13, wobei:
- der Bakterienstamm oder das Bakterium *Limosilactobacillus fermentum* I-5777 und/oder I-5778 in trockener, bevorzugt lyophilisierter Form vorliegt und in einer Menge von 1 x 10⁷ bis 1 x 10¹⁰ KBE, bevorzugt von 2 x 10⁸ bis 2 x 10⁹ KBE, und
- die Hefe Sc I-3856 in trockener Form vorliegt und in einer Menge von 1 x 10⁷ bis 1 x 10¹¹ KBE, bevorzugt von 1 x 10⁹ bis 1 x 10¹⁰ KBE und stärker bevorzugt von 2,5 x 10⁹ bis 5 x 10⁹ KBE enthalten ist.

## Claims

1. *Limosilactobacillus fermentum* strain chosen among the strain deposited with the CNCM on November 17, 2021 under number I-5777 or under number 1-5778, for its use in the prevention and/or treatment of vaginal candidiasis.

2. Bacteria obtained by culturing the *Limosilactobacillus fermentum* strain chosen among the strain deposited with the CNCM on November 17, 2021 under number I-5777 or under number I-5778, for its use in the prevention and/or treatment of vaginal candidiasis.

3. Bacteria for use according to claim 2, **characterized in that** it is presented in frozen form or in dry form, preferably in dry form, and more preferably still in freeze-dried dry form.

4. Strain for use according to claim 1 or bacteria for use according to claim 2 or 3, wherein the vaginal candidiasis is due to fungi chosen from the group comprising *Candida albicans, Candida tropicalis, Candida pseudotropicalis, Candida krusei, Candida glabrata,* and mixtures thereof.

5. Strain or bacteria for use according to any one of claims 1 to 4, wherein it is used in association with the *Saccharomyces cerevisiae* strain deposited with the CNCM on October 17, 2007 under number I-3856 and/or with the yeast obtained by culturing said *Saccharomyces cerevisiae* strain.

6. Strain or bacteria for use according to claim 5, wherein the yeast obtained by culturing the *Saccharomyces cerevisiae* strain is in the form of dry yeast or fresh yeast, and preferably in the form of dry yeast.

7. Strain or bacteria for use according to claim 6, wherein the dry yeast is in the form of active dry yeast or instant dry yeast.

8. Strain or bacteria for use according to claim 5, wherein the yeast is in the form of inactive yeast.

9. Strain or bacteria for use according to claim 5, wherein the yeast is in the form of a yeast derivative, said derivative being chosen among the cell wall of said yeast, the cell wall glucans of said yeast, the cell wall mannoproteins of said yeast, or mixtures thereof.

10. Composition comprising:
- *a Limosilactobacillus fermentum* strain chosen among the strain deposited with the CNCM on November 17, 2021 under number I-5777 or under number I-5778, and/or a bacteria obtained by culturing said *Limosilactobacillus fermentum* strain,
- the *Saccharomyces cerevisiae* strain deposited with the CNCM on October 17, 2007 under number I-3856 and/or the yeast obtained by culturing said *Saccharomyces cerevisiae* strain,
- possibly an excipient,
for its use in the prevention and/or treatment of vaginal candidiasis.

11. Composition for use according to claim 10, wherein the composition is a pharmaceutical composition, a food composition, or a food supplement.

12. Composition for use according to claim 10 or claim 11, wherein it is in a form suitable for oral or vaginal administration, preferably oral.

13. Composition for use according to any one of claims 10 to 12, further comprising an active ingredient chosen from the group comprising vitamin K, vitamin B9, glutathione, S-adenosyl-L-methionine (SAM-e), chondroitin sulfate, and mixtures thereof.

14. Composition for use according to any one of claims 10 to 13, wherein:
- *Limosilactobacillus fermentum* bacterial strain or bacteria I-5777 and/or I-5778 is in dry form, preferably freeze-dried, and is present in a quantity ranging from 1x10⁷ to 1x10¹⁰ CFU, preferably from 2x10⁸ to 2x10⁹ CFU, and
- yeast Sc 1-3856 is in dry form, and is present in a quantity ranging from 1x10⁷ to 1x10¹¹ CFU, preferably from 1x10⁹ to 1x10¹⁰ CFU, and even more preferably from 2.5x10⁹ to 5x10⁹ CFU.
